(19) Europäisches Patentamt
European Patent Office
Office européen des brevets

(11) **EP 4 382 122 A1**

(12) **EUROPEAN PATENT APPLICATION**

(43) Date of publication:
**12.06.2024 Bulletin 2024/24**

(21) Application number: **22020605.6**

(22) Date of filing: **08.12.2022**

(51) International Patent Classification (IPC):
*A61K 39/39* (2006.01)   *C07K 14/47* (2006.01)
*A61P 31/12* (2006.01)   *A61K 38/10* (2006.01)
*C07K 7/06* (2006.01)

(52) Cooperative Patent Classification (CPC):
**A61P 31/12; A61K 38/10; A61K 39/39; C07K 7/06;
C07K 14/47; C12N 9/6413;** A61K 2039/58;
C12N 2710/16634; C12N 2770/20034;
C12Y 304/23001

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Zhabilov, Harry H.
Frisco TX 75035 (US)**

• **Ivanov, Luchezar B.
Bankya (BG)**

(72) Inventors:
• **Zhabilov, Harry H.
Frisco TX 75035 (US)**
• **Ivanov, Luchezar B.
Bankya (BG)**

(74) Representative: **Stefanova, Stanislava Hristova
PO Box 70
1797 Sofia (BG)**

(54) **PEPTIDE COMPLEX IPF-H2A FOR MODULATION IMMUNE FUNCTION OF THE HUMAN BODY**

(57)   The invention provide a peptide complex IPF-H2A for modulation of the immune function of the human body and for antigen- specific immunotherapy. The peptide complex IPF-H2A, having SEQ ID: NO. 1, provides a new approach for treating immune disorders, viral diseases, cancer and immune related implications and also for antigen- specific immunotherapy

EP 4 382 122 A1

**Description**

**Field of the invention**

The present invention relates to a peptide complex IPF- H2A applicable for modulation of the immune function of the human body and for antigen- specific immunotherapy.

**Background of the invention**

Major Histocompatibility Complex (MHC) is a key component of T cell immunity. There are two types ofMHC-class I and class II. Professional antigen presenting cells (APC) include B cells, macrophages and microglia. B cells express MHC class II. Once antigen has been bound by the Ag receptor on the B cell, the Ag and receptor are engulfed into endosomal compartment. This compartment fuses with another compartment- lysosome. The B cells are efficient and breaking down Ag into smaller parts and loading the parts of MHC II in the lysosome. The MHC then trafficked to the cell surface where B cell "shows" the Ag to a CD4 T cell. CD4 cells are two categories- Th1 and Th2. B cell and CD4 cell encounter in the groove (antigen binding location in the MHC molecule) T cell receptor to recognize antigenic peptide by class I MCH on the surface of APC. Flexibility in both T-cell receptor's (TCR's and peptides plays key role in antigen recognition and discrimination. Flexibility of peptide binding groove impacting region contacted by TCR as well as other activating and inhibitory receptors of the immune system. Altered MHC flexibility will influence receptor engagement associated with receptor recognition. The "altered self" mechanism of immune recognition has implication for specificity cross-reactivity and antigenicity in cellular immunity (*W. F. Hawse- 6.8.2013*). Class I MHC proteins reflecting intrinsic class II MHC dynamics that could be modulated by peptides.

Peptides can modulate the flexibility of the MHC complex. Tuning of MHC dynamics by peptides reflects a fundamental mechanism for modulating the immune system. Single cell network profiling (SCNP) is a technology that quantifies functional immune signaling capacity and connectivity at a systems biology level.

The blocking of cellular receptors by chemical molecules or specific antibodies is possible. It has been recognized that denying entry into CD4+ cells to the HIV-1 virus could at least slow the progress of the infection and alleviate, if not cure, the disease and/or its symptoms. The complex mechanism by which the virus crosses the cell membrane has been widely investigated. Broadly, the entry of human immunodeficiency virus into, for example, CD4+ Th1 cells (T-helper type 1 cells), is dependent upon a sequential interaction of the gp120/gp41 subunits of the viral envelope glycoprotein gp160 with the CD4+Th1 cell surface glycoprotein and the cell surface receptor CCR5. On binding of gp120 with its cell surface binding sites, a conformational change in the latent gp41 subunit through an intermediate state to an active state is initiated, inducing fusion of the viral and cellular membranes and transport of the virus into the cell (Nature 387:426-30, 1997).

Accordingly, numerous binding experiments have been conducted in an effort to find antiviral ligands that will effectively compete with the HIV-1 for CD4+ gp and/or CCR5 binding sites, or that will preferentially block gp120 and/or gp41 binding domains. In one example, a reported structure (X-ray crystallography) comprising an HIV-1 gp120 core complexed with a two-domain fragment of human CD4 and an antigen-binding fragment of a neutralizing antibody that blocks chemokine-receptor binding, is said to reveal a CD4-gp120 interface, a conserved binding site for the chemokine receptor, evidence for a conformational change on CD4 binding, the nature of a CD4-induced antibody epitope, and specific mechanisms for viral immune evasion, "which should guide efforts to intervene" (Nature 393 (6686):632-1, 1998). Also, it has been shown that inhibition of the change in structure of gp41 from its intermediate to active state with peptides used as competitors for critical cell receptors may reduce viral load, and that while gp120 masks epitopes on the gp41 subunit in its latent state, gp41 may be vulnerable to neutralizing antibodies in its transient or intermediate state (Molecular Membrane Biology 16: 3-9, 1999). In another study, disclosed in patent US 2004/0018639, a protein designated "Thymus Factor" ("TF") is stated to have the ability to bind to a fragment of HIV-1 gp41 in gel electrophoresis, and that this binding property can be used to assay TF activity or in the treatment of HIV.

In addition, it is known by patent EP1830867 (A2) that the irreversibly inactivated pepsin fraction (IPF) having amino sequence GDEPLENYLDTEYF (-Gly-Asp-Glu-Pro-Leu-Glu-Asn-Tyr-Leu-Asp-Thr-Glu-Tyr-Phe-), effectively blocks gp41 and gp120 domains essential for viral entry into CD4+ cells and inhibit viral infection, in vivo and in vitro. According to the invention, this structure (IPF) is used to diagnose viral infection, particularly HIV-1 infection and as a prophylactic or therapeutic to prevent or to treat HIV infections. The inventor tested this compound for its ability to stimulate human natural killing T (NKT) cell lines, secretion of key cytokines such as IFN- IL2 and IL-12, and to activate autologous dendritic cells, as well as binding to CD1d and the invariant T-cell receptor. This protein exhibits a stronger adjuvant effect in various HIV vaccine platforms in mice. IPF also provides a protective adjuvant effect with a candidate HIV and HCV vaccine. While the majority of the studies performed focus on the potential of the IPF as a vaccine adjuvant, it is foreseeable that the compound could also be used as a potential immunotherapeutic to treat cancer, infectious disease, and autoimmune diseases.

The histones are conservative proteins and significant components in the chromatin structure of the cells. They are highly basic proteins rich in lysine and arginine residues and play an important role in various cellular functions such as immunity. There are five families of histones which are designated H1/H5 (linker histones), H2, H3, and H4 (core histones). Histones may be chemically modified through the action of enzymes to regulate gene transcription. The epigenetic modifications of histones contribute to their homeostatic functions of the cell, one of which is apoptosis. The research results of Williams, W. M. at al. (Clin Exp Immunol 1996, 104: 18-24) on the correlation between expression of antibodies of histone H2B and clinical activity in HIV- infected individuals, particularly those with symptomatic disease, revealed a significantly elevated titres of anti-H2B antibodies. Because the titres of H2B antibodies were found to parallel the numbers of circulated CD4 cells, the authors presume that they are intimately involved in the sequence of events that leads to the dysfunction and elimination of the helper/ inducer T lymphocyte population.

BRIEF DESCRIPTION OF THE DRAWINGS

Fig. 1 - Presents the binding of IPF-H2A complex to ovarian cancer human sera molecules

Fig. 2- Shows adjusted sensogram, presenting the specific signal in the positive 1:25 serum sample

Fig. 3- Shows image of uninfected and untreated cells (cell control) by atomic force microscopy (AFM)

Fig. 4- Shows the changes of the PPR signal

Fig. 5- Shows the differences in the kinetics of the antiviral activity of ACV and IPF-H2A

Fig. 6- Shows the effect of IPF-H2A complex on the survival/ proliferative activity of cells of Lep line accounted through MTT analysis after 96 hours of treatment

Fig. 7- Shows the protection (%) of *Lep* cells, contaminated with HCoV-229E and treated with the IPF-H2A complex and Tamiflu

Fig. 8- Shows the statistically significant survival differences between untreated control and treated with IPF-H2A complex cell cultures infected with HSV-1/ HSV-2

Fig. 9- Presents the inhibitory effects of IPF-H2A and of the anti-herpes preparation ACV

SUMMARY OF THE INVENTION

The invention provide a peptide complex IPF-H2A for modulation of the immune function of the human body and for antigen- specific immunotherapy. The peptide complex, according to the invention, provides a new approach for treating immune disorders, viral diseases, cancer and immune related implications and also for antigen- specific immunotherapy.

DETAILED DESCRIPTION OF THE INVENTION

According to one preferred embodiment of the present invention, the peptide complex IPF-H2A is a result of spontaneous binding (e.g. covalently bonded) of the isolated from lyophilized porcine Irreversible Pepsin Fraction (IPF) and the unfractionated whole Histone type 2-A (H2A). Thus, the binding of IPF structure having amino sequence GDEPLENYLDTEYF (SEQ ID: NO. 2) and the H2A structure having amino sequence NVPVSVEGYQITLDSIT (SEQ ID: NO. 3) leads to the structure, that of IPF-H2A peptide complex, representing a linear peptide with amino acid sequence GDEPLENYLDTEYFNVPVSVEGYQITLDSIT (SEQ ID: No. 1).

The Irreversible Pepsin Fraction (IPF) and the unfractionated whole Histone type 2-A (H2A) could be derived also from any other source, containing these sequences. For isolating and sequencing the components of IPF-H2A complex could be used common laboratory methods and reagents.

The peptide with amino acid sequence GDEPLENYLDTEYFNVPVSVEGYQITLDSIT (SEQ ID: No. 1) provides a novel mechanism to reduce viral burden and stimulate innate immune responses to the virus for patients with significant antiretroviral resistance; it appears to modulate helper T1 cells' expression of elaborate cytokines INFy, IL-2, which selectively promote cell- mediated immune response and subsequently stimulate cytotoxic lymphocytes. These lymphocytes have a prominent role in the host's immunologic response to viral infections. Proteins encoded by these pathogens enter the endogenous pathway for antigen presentation and are expressed on the surface of infected cell as a complex with class I MHC proteins. Peptide modulation of class I MHC in both TCR-s (T- cell receptor) plays a significant role in antigen recognition. Complex molecule of IPF-H2A and CD4 receptors reactivate immune system after activating Th1 Th2 cellular immune answer. Flexibility in both TCR's and peptides plays key role in antigen recognition and discrimination. Flexibility of peptide binding groove impacting region contacted by TCR as well as other activating and inhibitory receptors of the immune system. Altered MHC flexibility will influence receptor engagement associated withreceptor recognition. "Altered self" mechanism of immune recognition has implication for specificity cross-reactivity, and antigenicity in cellular immunity (*W. F. Hawse - 6.8.2013*).

Peptides can modulate the flexibility of the MHC complex. Tuning of MHC dynamics by peptides reflects a fundamental mechanism for modulating immune system. SCNP (*Single Cell Network Profiling*) plus CD4 complex form complex molecule reactivating Th1 cellular immune answer. Th1 cells secrete IL, IFN alpha, which are responsible for mediatingdilate type hypersensitivity reaction. Th2 cells secrete IL 2, IL4, IL5, IL6 and IL10, which are involved in, inducing humoral immune response. IPF-H2A peptide complex can modulate the flexibility of the region which dynamics reflects a fundamental mechanism for modulating immune response.

The blocking of cellular receptors by chemical molecules or specific antibodies is possible. The viralaggression uses one cellular receptor- CD4 and two co-receptors CCR5 and CXCR4. The first step in cell entry occurs when a glycoprotein on the viral surface (gp120) binds to surface receptors on the target cell. The next step is a new binding between envelop protein (for example HIV-1) and cell co-receptor CCR5. Once gp120 links to both receptors, gp41 (a trans-membrane viralcomplex) undergoes a conformational change and brings the viral membrane into proximity with the cell membrane. Fusion of two lipid delayers then occurs, thus allowing intracellular entry of viral contents.

It has been discovered in accordance with the present invention that the protein complex, named IPF-H2A, demonstrate *in vitro* equal binding with HIV-1 envelope protein gp41 (non-glycosylated fragment 579-601). The results are demonstrated on sensograms by *Biacore.* Binding responses RV- resonance units are illustrated for the protein immobilized surface in response to gp41, gp120, gp160 CD4.

Another preferred embodiment of the present invention is the immunomodulatory use of IPF-H2A complex and its use in the preparation of antigen- specific therapeutic vaccine, suitable for the treatment of several disorders including congenital defects involving primary T cell deficiency,viral infections, HIV, immunosuppression following cancer treatment, alone or in combination with antiviral disorders.

IPF-H2A peptide complex is preferably delivered with adjuvant, or is chemically conjugated to an immunogenic carrier that would provoke an antibody response in the patient. Examples of immunogenic carriers are keyhole limpet and bovine serum albumin.

In one aspect of the invention, IPF-H2A peptide complex is used to treat HIV infections, especially AIDS and ARC. For example, IPF-H2A is prepared for administration by mixing it at the desired degree of purity with adjuvant or physiologically acceptable carriers. Carriers, which are nontoxic to recipients at the dosages and concentrations are employed. Although IPF-H2A complex is desirably administered with an adjuvant, in situations where the initial inoculation is delivered with IPF-H2A complex, boosters with IPF-H2A may not require adjuvant. Injections (intramuscularly or subcutaneous) will be the primary route for therapeutic administration of the compositions of this invention. However, intravenous delivery, delivery through catheter, or other surgical tubing may also be used. Alternative routes include tablets and the like, liquid formulations, and inhalation of lyophilized or aerosolized receptors. Liquid formulations may be utilized after reconstitution from powder formulations.

IPF-H2A peptide complex could be administered also via microspheres, liposome is another micro particulate delivery systems or sustained release formulations placed in certain tissues including blood. The dosage of IPF-H2A peptide complex administered will depend upon the properties of the formulation employed. Its binding activity and *in vivo* plasma half-life, the concentration of IPF-H2A complex in the formulation, the administration route, the site and rate of dosage, the clinical tolerance of the patient involved, the pathological condition afflicting the patient and the like, are well within the skills of the physician. Different dosages are utilized during a series of sequential inoculations: the practitioner may administer an initial inoculation and then boost with smaller doses of IPF-H2A complex.

IPF-H2A peptide complex can be administered in several ways and to different classes of recipients. It can also be used to treat individuals who may or may not be at risk of exposure to HIV-1, and additionally, the IPF-H2A complex is desirably administered to seropositive individuals and to individuals who have been previously exposed to HIV-1. IPF-H2A peptide complex can be administered in combination with other antigens in a single inoculation "cocktail". IPF-H2A peptide complex can also be administered as in a series of inoculations administered over time. Such a series may include inoculation with the same or different preparations of HIV antigens or other vaccines. The adequacy of the treatment parameters chosen, e.g., dose, schedule, adjuvant choice and the like, is determined by taking aliquots of serum from the patient and assaying for Antibody and/or T cell titers during the treatment program. T cell titer may be monitored by conventional methods. For example, T lymphocytes can be detected by E-rosette formation as described in Bach, J.F., (Contemporary Topics in Immuno Biology, Vol. 2: Thymus Dependency, p. 189, Plenum Press, New York, 1973; Hoffman, T. & Kunkel, H. G., and Kaplan, M. E., et aL, both papers are in In Vitro Metlzo & in Cell Mediated and Tumor Immunity, B. R. Bloom & J. R. David Eds. Academic Press, New York (1976)). For example, the amount of T cell rosette formation may be assayed after the third but before the tenth week of IPF-H2A peptide complex treatment. An over sixty-five percent rosette formation indicates a good cell mediated immune response in the patient.

In addition, the clinical condition of the patient can be monitored for the desired effect, e.g., anti-infective effect. If inadequate anti-infective effect is achieved, then the patient can be boosted with further IPF-H2A peptide complex treatment and the treatment parameters can be modified, e.g., to potentate the immune response, such as by increasing the amount of IPF-H2A peptide complex and/or adjuvant, complexion IPF-H2A complex with a carrier or conjugating it to an immunogenic protein or varying the route of administration. IPF-H2A peptide complex may optionally be administered along with other pharmacological agents used to treat HIV-1 infections such as AIDS and ARC. Examples of these pharmacological agents are AZT, antibiotics, and immuno-modulators such as interferon, anti-inflammatory agents, and anti-tumor agents.

The examples presented below illustrate the invention without limitation its scope of protection.

**Example 1-** Preparation of IPF-H2A peptide complex

According to one preferred embodiment, the IPF-H2A peptide complex of the invention is prepared by spontaneous binding (e.g. covalently bonded) of **IPF,** isolated from active pepsin according to procedure described by Zhabilov, H. in patent US 7,479,538 B2 **and** the ready marketed unfractionated whole Histone type 2-A, in a ratio 10: 1. After preparation of the IPF-H2A complex, 5.6% aluminum adjuvant or IL2 is added.

The resulting IPF-H2A peptide structure represents a 42.4 kDa combined linear peptide with amino acid sequence GDEPLENYLDTEYFNVPVSVEGYQITLDSIT (SEQ ID: No. 1).

**Example 2-** Assessment of IPF-H2A Binding Activity using Surface Plasmon Resonance

The assessment of IPF-H2A activity was determined by Surface Plasmon Resonance (SPR) technology in Karolinska University Hospital, Stockholm, Sweden.

A receptor assay has been developed and validated to characterize the quality of different batches of IPF-H2A peptide complex preparations. Since biological activity always depends on a first binding step, the primary criterion for assessing biological activity is the ability of a compound to bind specifically to a ligand. The most sensitive biosensor instrument for detection compound-ligand interaction is the BIACORE. It directly captures any proteins including molecules from cytosolic or tissue extracts on sensor surface and measure binding kinetics with considerable ease and precision. It is widely used as the most sensitive method for measuring the active concentration of biomolecules and for their quality control.

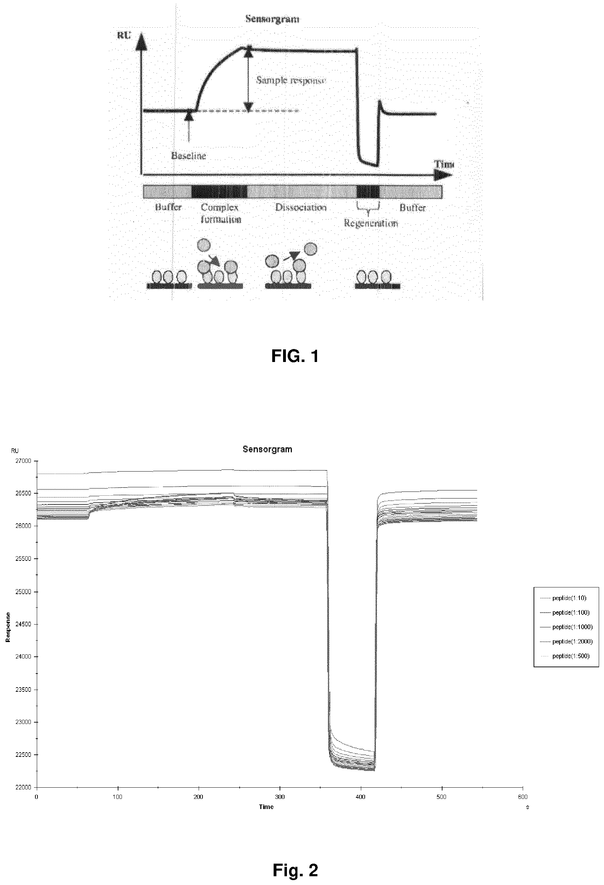

SPR is an optical technique that measures the refractive index change occurring at the sensor-fluidinterface layer. A plastic fluidics system plate is pressed into contact with a gold-coated glass chip (CM5), the surface of which is coated with an un-cross-linked carboxymethylated dextran polymermatrix. This allowed forming micro reaction chambers or flowing cells. The gold partial mirror is the optical port as well. Experiments are performed by a computer-program-driven robotics systemto facilitate consistency. The injection of analyte across a ligand immobilized in the matrix produces a real-time change in refractive index signifying an increase in associated molecular mass.In our work IPF-H2A preparation is the analyte, which binds to immobilized ovarian cancer human sera molecules. The data trace is a sensogram plotting response units (RU) against time **(Fig. 1).**

In the given assay it was measured the direct binding of an active component of IPF-H2A complex sample (currently unidentified biomolecule/s) to an immobilized human ovarian cancer sera molecule, which are binding for IPF-H2A complex. Immobilization of ovarian cancer human sera on the CM5 sensor surface was performed by standard amine coupling. The Biacore system was equilibrated in running buffer (PBS, 0.05% Tween 20, 1 m MEDTA, pH 8.4) at a flow rate of 5 ml/min. The carboxymethylated dextran matrix was activated by injection of 35 ml of a solution containing NHS (0.05 M)/ EDC (0.2 M) (50/50). Thirty-five microlitersof ovarian cancer human sera at 500 mg/ml in citrate buffer (pH 4; 0.01 M) was injected. The deactivation of the remaining NHS-ester groups was performed by injection of 35 ml of ethanolamine hydrochloride (pH 8.5; 1 M). A regeneration of the sensor surface was done by the injection of 5 ml of 1M NaCl and 0.1 M NaOH. The experiments were performed at a constant flow rate of 5 ml/min of running buffer. All the reagents were prepared by dilution in running buffer. Figure 2 shows kinetics of the specific binding and subsequent dissociation of IPF-H2A complex active component with immobilized ovarian cancer human sera. Maximal sample response (at the arrow) will be used in calculations and comparisons. The sensograms are performed on the Biacore system for one positive Ovarian cancer sample and one negative control where both samples have been diluted 1:25 and 1:100 for the serum: FC1 & FC2 are the negative sample diluted 1:25 and 1:100, respectively. FC3 & FC4 are the positive patient sample diluted the same. As could be seen on the sensogram of **Fig. 2,** a specific signal in the positive 1:25 serum sample was found that is significant and distinct from the negative control; app 100RU. The 1:10 and the 1:100 peptide seems to be the best dilution of the serum.

**Example 3-** Confirmation of the hypothesis of the inhibitory mechanism of action of an IPF-H2A peptide complex on the HSV-1 replication cycle

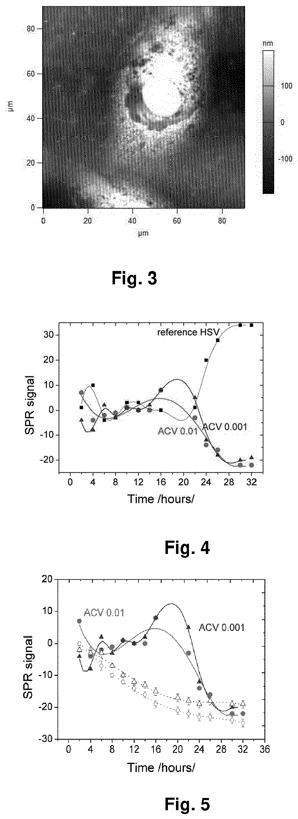

The inhibitory mechanism of action of an IPF-H2A peptide complex was confirmed on the HSV-1 replication cycle by establishing the kinetics of biomolecular host cell-virus interaction as well as IPF-H2A-treated infected cells under single-step replicationcycle. The study was performed by the Surface Plasmon Resonance (PPR) method activated on a gold-plated diffraction grating. As a model system in the tests was used a Vero monolayer cell line (from monkey kidney), where the cells were seeded on a biochip representing a gold-plated diffraction grating. The groove of the diffraction grating provides a tight cell/ surface contact, which is very important for correct measurements. **FIG. 3** shows an image of uninfected and untreated cells (cell control) by atomic force microscopy (AFM). The seeding density of the cells was controlled so that several thousand cells determined the changes in plasmon resonance.

As a viral model in antiviral experiment were used HSV-1 cells. As a positive control in antiviral experiments, the widely used antiviral drug was used Acyclovir- (2-amino-1,9-dihydro-9-((2-hydroxyethoxy)methyl)-6H-purin-6-one), ACV) (*Sigma-Aldrich, Germany*). Measurements were made on every 2 hours until the 32-nd hour of viral infection development, the last hours coinciding with the initial cytopathic cellular changes. As a rule, the increase/ decrease of the PPR signal is a consequence of the increase/ decrease of the effective index of refraction of the cell monolayer as a result of the morphological changes in the cells caused in the process of viral replication. The stages of the replication cycle- adsorption, penetration of the virus into the cell, changes in cellular metabolism, as well as the assemblyand release of the daughter offspring, cause such changes and accordingly generate a PPR signal. The changes of the PPR signal are shown in **Fig. 4** where the inhibitory effect of ACV-treated cells under conditions of a single-cycle test for HSV-1 development: viral control (HCV-infected cells at MOI = 1); Infected and treated with ACV cells: at ACVconcentration = 0.001 mg/mL (MNC) - at ACV concentration = 0.01 mg / mL and in the **Fig. 5,** showing the differences in the kinetics of the antiviral activity of ACV and IPF-H2A.

Obviously, IPF-H2A acts at the time of virus penetration into the cell. There is no characteristic increase in the signal in the range of 8-16 hours associated with the presence of viral capsids in the cytoplasm, which indicates the absence of virus. The difference in PPR signals becomes clear after the 14-th hour. This completely coincides with the inhibition of the process of viral replication, which corresponds to the mechanism of action of the drug. After the 20th hour, there is no increase in the SPR signal, as there are no newly created virions.

**Example 4:** Immunological examination of coded blood samples of patients with HIV-1

For the period 02.11.2015-14.05.2016 in the *Specialized Hospital of Active Treatment of Infectious and Parasitic Diseases "Prof. I. Kirov", Sofia,* were performed totally 115 immunological examinations and the indices are reported as follows: absolute number (AN) of the general lymphocytes, percentage, and absolute number of T (CD3+) lymphocytes, B (CD19+) lymphocytes and NK (natural killers) (CD4+CD3+) cells, percentage, and absolute number of T-lymphocyte subpopulations CD4 T (CD4+CD3+), CD8 T (CD8+CD3+) as well as their ratio (CD4/CD8).

Complete follow-up course (three visits) has been done in 31 patients. Nine have given up after the first examination; 5 (five) patients have two examinations. There are also 3 (three) examinations done out of schedule. The results of the first, second and third examinations of patients with complete follow-up course are summarized. The results of all performed examinations are presented in Table 1 below.

*Results of the baseline examinations*

The average (min- max) values of the examined populations are summarized on Table 1. The absolute number of CD 4 T lymphocytes is (average, min-max) 528 (116-1261)cells/$\mu$l and the percentage is 22 (4-44)%. The distribution of patients according to the degree of immune deficiency at the baseline examination is as follows: 9% (n = 3) with severe immune deficiency (CD4 AC < 200); 32% (n = 10) with initial to moderate immune deficiency (CD4 AC 200 - 400) and 59% (n=18) without manifested immune deficiency (CD4 AC > 400). Respectively in the greater part of the patients (81%, n = 25) the CD8 Tcells are increased and the CD4/CD8 index is decreased below 0.9 (93.5%, n = 29).

From the rest indices: lymphocyte number (average, min- max) 2.379 (0.604- 4.835) with deviation from normal in 22.5% (7 persons); 2 persons with decreased lymphocyte number (< 900) and 5 persons with lymphocytosis (> 3100); B cells (average, min-max) 0.221 (0.044-0.425) with deviation from normal in 71%, respectively: absolute decrease in52% (n = 16); relative decrease in 19% (n = 6); NK cells (average, min- max) 0.341 (0.07- 0.846) with deviation from the absolute number in 10% (n = 3), respectively- below the lower reference limit in two and above the upper reference limit in one patient; the relative share is decreased in one patient and is above the upper limit- in two.

*Results of the second examination*

The results are summarized in Table 1. It is observed increase of the absolute number and the relative share of: B cells (Wilcoxon's $p < 0.05$) and CD4 T subpopulation (Wilcoxon'sp $< 0.01$). At the same time, the share of CD8 T lymphocytes decreases ($p < 0.01$) which is reflected by the statistically significant increase of the CD4/CD8 index ($p < 0.01$). At the end of the 8th week in the category without manifested immune deficiency (CD4 AC > 400) are 71% of the examined patients (n = 22).

Table 1

| | ABSOLUTE NUMBER | | | | | | PERCENT | | | | | |
|---|---|---|---|---|---|---|---|---|---|---|---|---|
| | Ly | CD3 T | CD4 T | CD8 T | B Ly | NK | CD3 T | CD4 T | CD8 T | B Ly | NK | CD4/CD8 index |
| FIRST EXAMINATION | | | | | | | | | | | | |
| AVERAGE | 2. 379 | 1.802 | 0.473 | 1.238 | 0.221 | 0.341 | 74.3 | 20.9 | 49.9 | 10,6 | 15.0 | 0.47 |
| MAX | 4.835 | 4.276 | 1.261 | 2.818 | 0.425 | 0.846 | 88.9 | 42.7 | 74.7 | 20.0 | 44.0 | 1.23 |
| MIN | 0.604 | 0.477 | 0.116 | 0.209 | 0.044 | 0.070 | 42.0 | 3.9 | 23.9 | 2.5 | 3.8 | 0.06 |
| P5 | 0.824 | 0.665 | 0.120 | 0.381 | 0.048 | 0.076 | 44.1 | 4.6 | 25.2 | 3.0 | 5.4 | 0.07 |
| P95 | 4.810 | 4.081 | 1.103 | 2.810 | 0.422 | 0.774 | 88.6 | 38.7 | 722.1 | 20.0 | 38.1 | 1.07 |
| SECOND EXAMINATION | | | | | | | | | | | | |
| AVERAGE | 2.443 | 1.839 | 0.530 | 1.210 | 0.254 | 0.338 | 74.29 | 22.35 | 48.16 | 10.86 | 14.22 | 0.51 |
| MAX | 4.075 | 3.273 | 0.966 | 2.708 | 0.486 | 0.642 | 87.76 | 38.01 | 71.93 | 20.08 | 32.33 | 1.01 |
| MIN | 1.094 | 0.631 | 0.122 | 0.350 | 0.069 | 0.093 | 44.42 | 5.49 | 24.63 | 2.79 | 4.77 | 0.08 |
| P5 | 1.251 | 0.728 | 0.148 | 0.450 | 0.086 | 0.101 | 49.33 | 5.86 | 25.60 | 3.39 | 6.11 | 0.09 |
| P95 | 4.053 | 3.165 | 0.915 | 2.378 | 0.479 | 0.637 | 87.48 | 37.45 | 71.40 | 19.33 | 29.00 | 1.01 |
| THIRD EXAMINATION | | | | | | | | | | | | |
| AVERAGE | 2.560 | 1.923 | 0.581 | 1.217 | 0.263 | 0.352 | 74.0 | 23.3 | 46.2 | 10.8 | 14.3 | 0.56 |
| MAX | 4.189 | 3.638 | 1.232 | 2.926 | 0.505 | 0.876 | 89.1 | 44.3 | 71.7 | 20.4 | 45.8 | 1.34 |
| MIN | 1.300 | 0.761 | 0.160 | 0.425 | 0.073 | 0.064 | 39.8 | 5.3 | 22.3 | 3.3 | 3.8 | 0.09 |
| P5 | 1.433 | 1.034 | 0.208 | 0.521 | 0.112 | 0.111 | 54.8 | 8.9 | 29.1 | 4.2 | 5.5 | 0.13 |
| P95 | 4.045 | 3.329 | 1.093 | 2.059 | 0.458 | 0.716 | 87.2 | 36.6 | 67.6 | 18.5 | 31.1 | 0.97 |
| Reference values | | | | | | | | | | | | |
| AVERAGE | 1,800 | 1,200 | 0,700 | 0,400 | 0,200 | 0,300 | 72 | 44 | 24 | 18 | 12 | 1,90 |

In practice in 17 patients (55% of the examined) is observed increase of CD4AC. In 9 of them (29% of the examined) it is accompanied by increase of the relative share of CD4and the CD4/CD8 index that corresponds to decrease of the immune activation. In four patients (13% of the examined) is reported decrease of CD4. Only in one patient the decrease of CD4 AC is accompanied also by decrease of the percentage of CD4 cells and the CD4/CD8 index. In the rest 10 patients (29% of the examined) there is no significant change in the absolute number of CD4 in comparison with baseline examination. In three of them is observed increase of CD4% and CD4/CD8 index.

*Results of the third examination*

The results are summarized in Table 1. In comparison with the baseline examination are observed: increase of the absolute number and the relative share of B cells (respectively p< 0.05 and P< 0.01) and of CD4 T subpopulation (p< 0.001). At the same time, the share of CD8 T lymphocytes decreases (p< 0.001) which is reflected by the statistically significant increase of the CD4/CD8 index (p< 0.001).

In 21 of the examined patients (68%) is observed increase of the absolute number of CD4 in comparison with the baseline examination. In 16 of them the increase of the absolute number of CD4 is accompanied by increase of the CD4/CD8 index and/or CD4% that corresponds to decrease of the immune activation. In the rest 10 patients (32%) on the third examination are not found significant changes in the absolute number of CD4. In comparison with the baseline values on the third examination, there are no patients with decrease of the percentage of CD4 cells or of the CD4/CD8 index.

**Example 5.** Examination of the antiviral effect of IPF-H2A complex against human corona virus 229E strain (HCoV-229E) in cell cultures

Currently in medical practice are applied 50 antiviral preparations as most of them are used for treatment and prevention of diseases caused by some viruses including respiratory. Despite of significant successes of chemotherapy, cannot be considered that at the present stage of treatment of viral diseases have been achieved any results. Low selectivity, significant toxicity, occurrence of resistant mutants, high mortality rate and occurrence of new zooantroponotic viruses are the basic reason for searching new antiviral means.

Coronaviruses (*Coronaviridae*) are family of a single chain RNA viruses. In January 2020, the family comprises 40 species combined in two subfamilies: *Orthocoronavirinae* and *Letovirinae.* Most of them infect animals like bats, chickens, camels, and cats. Sometimes viruses who infect one species may mutate in such a way that they become able to infect another species. That's so called "cross-transmission of species" or "overflow". To date seven coronaviruses can cause diseases in humans. They belong to *Orthocoronavirinae* subfamily. Coronaviruses (CoV) cause diseases that vary from mild cold (HCoV-NL63, HCoV-229E, HCoV-OC43 and HCoV-HKU1) to more severe diseases likeMiddle East respiratory syndrome (MERS- CoV), severe acute respiratory syndrome (SARS-CoV) and the new Covid-19 (SARS-CoV-2). Coronaviruses HCoV-229E, -NL63, -OC43-HKU1 constantly circulate in human population and cause respiratory infections with basic symptoms fever and sore throat, swollen lymph nodes, bronchitis, rarely pneumonia in winter and early spring in adults and children. They rarely are with fatal outcome. There are no vaccines or antiviral medicines that have been approved for prophylaxis or treatment of corona viruses.

The study analyzed the antiproliferative and potential antiviral properties of the IPF-H2A complex, according to the present invention, against human coronavirus (HCoV-229E strain) in cell cultures.

The study provides dissolving of IPF-H2A peptide complex to initial concentration of 5 mg/ml (starting solution), from which *ex tempore* are prepared series solutions with nutritional medium DMEM (Dulbecco's Modified Eagle Medium, Sigma- Aldrich, Germany), containing 2% fetal beef serum (FBS, Gibco) and 1% antibiotic solution (100 U/ml penicillin and 100 $\mu$g/ml streptomycin sulphate (Gibco, USA)) (operational solutions), that are filtrated throughsterile antibacterial filters with pore diameters 0.45 $\mu$m (Sartorius Stadium Biotech, Australia).

As a model system is used monolayer of human embryonic lung (Lep) cell line. The cells are cultivated in DMEM with added thermally inactivated 10% FBS, 1% Sodium pyruvate (Sigma-Aldrich, Germany) and antibiotics (penicillin- 100 U/ml), streptomycin sulphate (100 $\mu$g/ml) (growth medium) and the cell cultures are incubated at 37°C in 5% CO2 and in the presence of the required humidity.

In the antiviral experiments has been used the reference strain of human coronavirus HCoV-229E, collection of "Robert Koch" Institute, Germany. The viral suspension is cultivated in maintenance nutritional medium DMEM, containing 2% FBS, 1% antibiotic solution (100 U/ml penicillin G sodium and 100 $\mu$g/ml streptomycin sulphate). The infectious viral titer is accounted in two methods: 1. Reed and Muench method of final dissolutions (1938) and is expressed as TCID 50 (50% Tissue Culture Infectious Dose) (reciprocal viraldissolution in which 50% of the cell culture is infected) and 2. colorimetrical MTT test. After triple freezing and thawing the obtained virus-containing material is centrifuged (2500 rpm/min, 15 min at 4°C), aliquoted and frozen at -80°C.

As a positive control in antiviral experiments is used oseltamivir phosphate (trade name Tamiflu). Each capsule contains 75 mg oseltamivir phosphate, corresponding to 75 mg oseltamivir, that initially is dissolved in PBS (stock solution with initial concentration 20 mg/ml) which serves for preparation of series operational solutions.

Cell freezing and thawing has been performed according to the generally accepted methods as described by *Pegg D., 2007.* The cells are frozen in concentration at least $1 \times 10^6$ cells/ml. During the cell thawing the ampoules are quickly put in a vessel with warmed to 37-40°C water and after thawing they are immediately transferred in a nutritional medium containing 10% FBS. Aiming prevention of the toxic effect of DMSO, the nutritional medium is substituted by new immediately after the cell adhesion to the padding (not later than 20 hours after seeding).

*Antiviral experiments*

*Determination of infectious titer through Reed-Muench method*

Immediately before work are prepared tenfold logarithmic dilutions of the relevant viral strain in maintenance nutritional medium DMEM, containing 2% FBS, 1% antibiotic solution (100 U/ml penicillin G sodium and 100 $\mu$g/ml streptomycin sulphate). When the cell monolayer reaches between 70- 80% confluence (concentration $5 \times 10^3$ cells/well, commonly after 24 hours) the nutritional medium in the wells is removed.

The cell monolayer is contaminated with the prepared viral dilutions in volume 0.1 ml. With each viral dilution are contaminated 4 wells. The virus adsorbs for 1 hour in a thermostat at temperature 33°C, thereafter to each well are added 0.1 ml maintenance nutritional medium. For cell control serve four wells in which are placed uncontaminated cells. The processed in this way plate is incubated in a thermostat at temperature 33°C and the required humidity. In occurrence of cytopathic effect (CPE) (occurrence of rounded cells irregularly disseminated in the cell monolayer tending to formation of foci) is waiting till development of 2 -3 + (50 - 75% of the cell monolayer) or till the process stops. The viral titer is expressed as $TCID_{50}$ (50% Tissue Culture Infectious Dose). The presence or the absence of viral cytopathic effect is assessed according to the following formula:

T = (logarithm of the dilution at which the % of contamination is > 50%) + (reciprocity factor x logarithm of the dilution factor).

Proportionality factor = [(% of contamination is > 50%) – 50%] / [(% of contamination is > 50%) – (% of contamination is < 50%)].

*CPE inhibition test. Determination of inhibitory concentration 50% ($IC_{50}$) and selectivity index (SI)*

The antiviral action of the tested substance is determined by *Mosmann* MTT test for determination the of cell survival, modified by *Shigeta S.* for quick screening for anti-influenza effect of compounds under the influence of the viral cytopathic effect the cells die. The determination of cell viability through MTT test of contaminated with virus and treated with the substance cells is indicative for the antiviral effect of the substance itself.

Twenty-four- hour monolayer Lep cultures, seeded in 96-well plates (with density $5 \times 10^3$ cells/well) are inoculated with tenfold logarithmic HCoV-229E dilutions, starting from $10^{-1}$ to $10^{-8}$ included, as with each dilution are contaminated 4 wells. The volume of the inoculated in a well viral suspension is 0.1 ml, in operational dose 100 $TCID_{50}$. The virus adsorbs for 1 h in a thermostat at temperature 33°C, thereafter to each well is added 0.1 ml maintenance nutritional medium. During the viral adsorption are prepared dilutions of the tested substance. After the end of the viral adsorption the plate is processed as follows:

Cell control (uncontaminated with virus and not treated cells) - to the designed for cell control wells is dripped 0.1 ml maintenance nutritional medium.

Influenced cells - (cells contaminated with virus and treated with various dilutions of the tested substance) -in wells are dripped 0.1 ml of the preliminary prepared dilutions of the tested substance.

The cell cultures are incubated in a thermostat at 33°C 5% $CO_2$ and are observed every day under light microscope for development of CPE. The effect of the action of IPFH2-A is accounted after the contamination (*p.i.*). The infectious titer is calculated according to Reed and Muench method. After accounting the results under microscope, the medium is ravaged, the plates are once washed with 0.2 ml/well PBS (pH = 7.2), thereafter the cell survival is determined through MTT test as described above. The IPFH2-A activity is expressed as % protection and is calculated according to the formula: [CDS - ODVC] % CPE = $\times$ 100 where:

$OD_S$ - Optic density of cells contaminated with virus and treated with various dilutions of the tested substance;

$OD_{VC}$ - Optic density of contaminated with virus cells (virus control)

The calculations are performed through the programs *Graph Pad Prizm* software version 8.00 *for Windows, LaJolla California USA and Origin 6.1* ™.

Inhibitory concentration 50% ($IC_{50}$) is the concentration of the test substance that inhibits with 50% the virusinduced cytopathic effect as is directly determined from the "dose-response" curve.

The selectivity index (SI) is calculated based on the $CD_{50}/IC_{50}$ ratio. $SI = CD_{50}/IC_{50}$.

*Statistical processing of the obtained results*

The obtained results are presented through mean values $\pm$ standard error of the mean (SEM) from at least two separate experiments, every one performed in three to five reps. Statistically significant differences in survival/proliferation between the untreated controls and the treated with tests in different concentrations wereassessed through ANOVA unifactorial dispersion analysis and subsequent *Dunnett* test. Statistically significant differences between the untreated controls and the treated samples have been assessed through t-test as the values at $p < 0.05$ are defined as statistically significant.

*Effect of inactivated pepsin fragment (IPFH2-A complex) on the survival and proliferative activity of cultivated in laboratory conditions cells*

The first important stage of the antiviral experiments includes determination of the cytotoxicity of the tested substance on *Lep line* cells, cultivated in laboratory conditions.

MTT analysis of the cell survival and proliferation was performed in a wide range of concentrations of the tested substance. The limits within which varied the applied concentrations have been selected on the grounds of preliminary experiments that included larger concentration intervals in order more precise determination of $CD_{50}$ and MNC values. The test substance is applied in concentration range 0.0001- 1 mg/ml, while the anti-nfluenza medicine Tamiflu® - 0.001-7.5 mg/ml for 96 hours. The untreated *Lep cells* whose survival was accepted as 100%, were used as a negative control. From the applied MTT test are obtained dose-response curves of the influence of the applied IPF-H2A complex on the cell monolayer. The analysis of the cell morphology surveillance is synchronized with the MTT analysis of the cell surveillance.

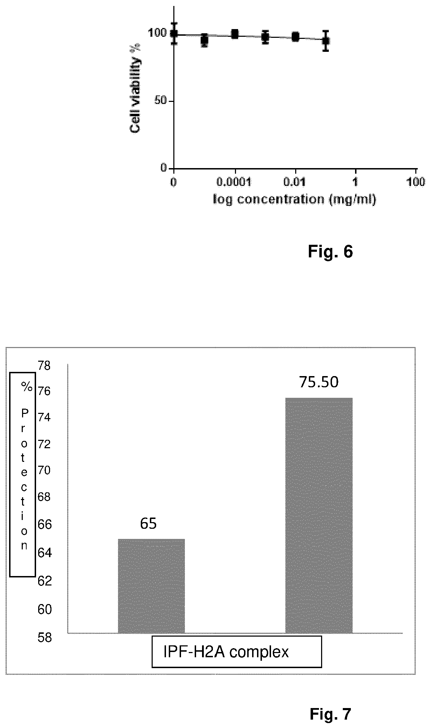

The obtained results show that the general survival of the treated monolayer of the Lep line is not drasticallydecreased under the influence of the tested inactivated pepsin fragment (IPFH2-A complex), unlike under the effect of the antiviral medicine Tamiflu. Like in Tamiflu the observed effect increases in direct proportion with the increase of its concentration. In the lowest tested incubational concentrations (0.0001-0.1 mg/ml) is observed mild survival inhibition (85.34- 99.52 %), while in the subsequent increasing concentrations (from 1 mg/ml to 2.5 mg/ml) the cell survival rate is not decreased and does not reach rates near to 50%. The differences between the survival in the untreated control and treated sample are statisticallysignificant in the applied doses of 0.0001 mg/ml ($p < 0.001$) and of 1 mg/ml ($p < 0.05$) **(Fig. 6).** Based on their cytotoxicity the tested substances are ranked in the following hierarchical sequence: Pursuant MNC: Tamiflu > IPF-H2A complex.

Comparing the experimental data for cell survival it becomes clear that the widely applied anti-influenza medicine Tamiflu is twentyfold more toxic than IPF-H2A peptide complex (Figure 6 and Table 2).

In conclusion, after the 96th hour from the treatment, the tested IPFH2-A complex displays no toxicity on cells of *Lep* line.

The decrease of the number adherent viable cells as well their rounding and reduce of size follow the trend found by the MTT analysis and is shown on Table 2 below.

Table 2

| IPF-H2A and Tamiflu® | Method | MNC mg/ml | $CD_{50}$ |
|---|---|---|---|
| IPF-H2A complex | Microscopic surveillance of the changes in the morphology of cell monolayer | 0.1 $\pm$ 0.1 | No data |
| | MTT test | 0.1 $\pm$ 0.12 | No data |

(continued)

| IPF-H2A and Tamiflu® | Method | MNC mg/ml | CD$_{50}$ |
|---|---|---|---|
| Tamiflu® | Microscopic surveillance of the changes in the morphology of cell monolayer | 0.001 ± 0.6 | 2.5 ±0.02 |
| | MMT test | 0.005 ± 0.15 | 2.5 ±0.06 |

*Antiviral activity*

*CPE inhibition test*

The IPF-H2A complex has been examined for antiviral effect on HCoV-229E in cell cultures. For testing the effect of the examined substance IPF-H2A complex on the HCoV-229E replication have been applied concentrations within the limits from 0.001 mg/ml to 1 mg/ml (MNC and close to it). For positive control in the antiviral experiments has been used oseltamivir phosphate (Tamiflu), applied in concentration range 2.5 mg/ml. For defining the inhibitory concentration leading to 50% inhibitionof the viral cytopathic effect (IC$_{50}$) have been drawn dose-response curves.

Initially the antiviral effect of the tested substance IPF-H2A peptide complex has been assessed through CPE inhibitiontest. After cell contamination with HCoV-229E the CPE occurs after approximately 96 hours and is expressed with occurrence of rounded cells irregularly disseminated in the cell monolayer tending to form foci. The cell cultures are daily observed for CPE development under light microscope. The effect of the action of the tested substances is accounted 96 hours (the time of the replication cycle of coronaviruses) after Lep cell contamination with HCoV-229E (p.i.). The results of the performed experiments are assessed in comparison with cells uncontaminated with virus and without addition of substance (cell control) as well as with cells contaminated with human coronavirus 229E strain without addition of substance (virus control).

As a positive control has been used a medicine with proven antiviral effect (in our case Tamiflu - applied in the clinical practice neuraminidase inhibitor of the influenza virus).

The study showed clearly that unlike the uncontaminated Lep cells, the contaminated withHCoV-229E cells are with a typical rounded shape, clustered like "islands", isolated each of other and separated from the padding on which they are cultivated.

The best inhibition of CPE of HCoV-229E, is observed after treatment of the contaminated Lep cells with IPF-H2A complex. Less expressed CPE inhibition but closer to the caused by IPF-H2A complex was found in the antiviral medicine Tamiflu®. The titer of the infectious viral yield is significantly reduced when the contaminated Lep cells have been treated with IPF-H2A complex, according to the present invention.

*Determining the effect of the tested substance on the infectious viral yield. Determination of the inhibitory concentrations 50% (IC$_{50}$) and the selectivity index (SI)*

For determination of the anti-HCOV-229E action of the tested IPF-H2A peptide complex, concentrations close to the minimum effective (0.0001 - 1 mg/ml) were applied.

The samples for accounting extracellular virus have been titrated in 24 hours Lep cell's monolayer in 96-well plates with accounting of the infectious viral titer in *log10*, according to *Reed* & *Muench* method of final dissolutions.The differences between in the infectious viral titers of the experimental and control samples have been expressed as *Δlog*. Based on the results are drawn "dose-response" curves and are defined minimum inhibitory concentrations 50 (MIC$_{50}$). For reliable result in the performed tests are accepted $\Delta log10_{values} \geq 1$.

Based on the obtained data "dose - anti-HCoV-229E" it was found that applied alone every one of the tested substances inhibits HCoV-229E replication in a dose- response way.

The inactivated pepsin fragment (IPF-H2A complex) effectively inhibits the infectious viral yield in concentration range 0.001-1 mg/ml. Applied in MNC (0.1 mg/ml) it reduces the viral yield with $\Delta log_{10}$ 1.3, asits MIC$_{50}$ is 0.01 mg/ml (Table 3). Despite that, the anti- influenza medicine Tamiflu effectively inhibits HCoV-229E replication in larger concentration range 0.00001-1 mg/ml (Table 4).

Table 3

| Viral strain | Concentration (mg/ml) | Viral yield ($log_{10}$ $CID_{50}$)* | $\triangle log_{10}$ |
|---|---|---|---|
| HCoV-229E | 0 | 5.833 ± 0.000 | - |
| | 0.001 | 4.498 ± 0.763 | 1.3 |
| | 0.01 | 4.296 ± 0.500 | 1.3 |
| | 0.1 | 4.000 ± 0.363 | 1.3 |
| | 1.0 | 4.251 ± 0.288 | 1.3 |
| *Data are presented with mean values ± SEM | | | |

Table 4

| Viral strain | Concentration (mg/ml) | Viral yield ($log_{10}$ $CID_{50}$)* | $\triangle log_{10}$ |
|---|---|---|---|
| HCoV-229E | 0 | 5.300 ± 0.000 | - |
| | 0.0001 | 4.562 ± 0.288 | 0.8 |
| | 0.001 | 4.000 ± 0.500 | 1.3 |
| | 0.01 | 4.124 ± 0.083 | 1.3 |
| | 0.1 | 4.000 ± 0.763 | 1.3 |
| | 1.0 | 3.515 ± 0.333 | 1.8 |
| *Data are presented with mean values ± SEM | | | |

Inhibitory concentration 50% ($IC_{50}$) is the concentration of the tested substance that inhibits with 50% the virus-induced cytopathic effect as it is directly determined from the "dose-response" curve. The principle chemotherapeutic indicators $IC_{50}$ of the tested substances are accounted based on the experimental results. The results according to $IC_{50}$ allow a hierarchical scheme to be drawn as follows: For HCoV-229: Tamiflu® > IPF-H2A peptide complex

The protection (%) of Lep cells, contaminated with HCoV-229E and treated with the IPF -H2A peptide complex and Tamiflu, applied in MNC is illustrated on **Fig.7.**

**Example 6:** Investigation of Antiviral Effects of IPF-H2A complex against human Herpes Simplex Viruses Type 1 & Type 2 (HSV-1/ HSV-2) in cell culture

It is known that for 90-95% of the adult population there is serological evidence of at least one HSV infection. A common feature of herpes viruses is that after having infected the host they cause a latent, chronic infection. Under the influence of stress factors, the latent herpes virus gets reactivated, causing diseases with an extremely diverse clinical symptoms- from asymptomatic infections and self-limiting, transient labial and genital lesions (HSV-1 and HSV-2), through retinitis, keratitis, keratoconjunctivitis (HSV-1, HSV-2, CMV), to severe generalized infections, encephalitis, meningitis, including malignant neoplasms (HSV-1/2, EBV, HHV-8). The importance of herpes infections as a concomitant disease and as a factor in the aggravated clinical symptoms in patients with AIDS has increased in recent years. In many cases, the lethal outcome for these patients is the result of a herpes relapse.

Despite the availability of highly effective therapy with drugs from the group of the nucleoside analogues in clinical practice, the need for new and effective drugs is becoming increasingly urgent. The main reason for this is the rapidly developing resistance of viruses to this class of drugs, with immunocompromised patients being particularly severely affected.

For analysis of the anti-proliferative and potentially antiviral properties of the IPF-H2A complex, according to the present invention, in cell culture, IPF-H2A was dissolved to an initial concentration of 50 mg/ml (background solution). Using the solution, a series of dilutions are done *ex tempore* with a nutrient medium DMEM (*Dulbecco's Modified Eagle Medium, Sigma-Aldrich, Germany*), which includes 2% fetal bovine serum (FBS, Gibco) and 1% antibiotic solution (100 U/ml penicillin and 100 µg/ml streptomycin sulphate *(Gibco, USA)*) (working solutions), which solutions are then filtered through sterile antibacterial filters with a pore diameter of 0.45 µm. As a model system in the tests was used a kidney monolayer cell line of African Green Monkey (*Cercopithecus aethiops*).

Cells were cultured on DMEM nutrient medium with termoinactivated 10% FBS, 1% sodium pyruvate (*Sigma-Aldrich, Germany*) and antibiotics (penicillin (100 U/ml), streptomycin sulphate (100 µg/ml) (growingmedium). The cell cultures were incubated at 37 °C at 5% CO2 and the required humidity. The blending of the Vero cells was done at 1:3 - 1:5 at a density of about $2 \times 10^5$ cells/ml, then resuspended several times and transferred onto cell culture mattresses ($25\ cm^2$). Before inoculating the cells with virus and/or IPF-H2A dilutions, the monolayer was washed three times with phosphate-buffered saline (PBS) with pH 7.4 for 1- 2 minutes, after which the solution was removed. All experiments were performed during the exponential phase of cell growth.

In the antiviral experiments were used two ACV-sensitive strains: Herpes Simplex virus type 1 (HSV-1) and type 2 (HSV-2), and Victoria strain (HSV -1) and strain Bja (HSV -2).

Viral strains were cultured in DMEM nutrient medium containing 2% FBS,1% antibiotic solution (100 U/ml penicillin G sodium and 100 $\mu$g/ml streptomycin sulphate).

Infectious virus titer was account for, also by Reed and Muench's method of final dilutions, and expressed as $TCID_{50}$ (50% Tissue Culture Infectious Dose) and also quantitatively through the colorimetric MTT method. After triple freezing and thawing, the resulting virus- containing was centrifuged (2500 rpm/min, 15 min at 4 °C), aliquoted and frozen at -80°C. As a positive control in antiviral experiments was used Acyclovir 2-amino-1 ,9-dihydro-9-[(2-hydroxyethoxy)methyl]-6H-purin-6-one (ACV) (Sigma- Aldrich, Germany), which was initially dissolved in PBS (stock solution with a background concentration of 1 mg/mL), and it was used for a series of working dilutions.

After blending the culture, conducting a Trypan Blue Dye Exclusion Test, freezing and thawing, the cells are transferred to a nutrient medium containing 10% FBS and proceeded to determination of the cell viability. The latter is conducted by a Microscopic observation of morphological changes in the monolayer of treated cells and Colorimetric MTT analysis according to *Mosmann,* 1983, with minor modifications.

*Antivirus Experiments*

*Determination of Infectious Viral Titer by Reed-Muench Method*

Immediately prior to work, ten-fold logarithmic dilutions of the relevant virus strain were prepared in DMEM nutrient medium containing 2% FBS, 1% antibiotic solution (100 U/mL penicillin G sodium and 100 $\mu$g/mL streptomycin sulphate). When the cell monolayer reached between 70- 80% confluence (concentration $5 \times 10^3$ cells/well, usually after 24 h), the nutrient medium was removed from the wells. The cell monolayer was infected with the prepared viral dilutions of 0.1 mL. With each viral dilution, 4 wells were infected. The virus was adsorbed for 1 h ina thermostat at a temperature of 37°C, then to each well was added 0.1 mL of nutrient medium. Four wells containing uninfected cells were used for cell control. Thus treated plate was incubated in a thermostat at a temperature of 37°C and at the required humidity. In the event of a cytopathic effect (CPE) (appearance of round cells, unevenly disseminated in the cell monolayer, with a tendency to formfoci), it was waited until the development of 2-3 + (50 - 75% of the cell monolayer) or until it stopped progressing. The viral infection titer is expressed as $TCID_{50}$ (50% Tissue Culture Infectious Dose). The presence or absence of a viral cytopathic effect is determined by the following formula:

$$T = (\text{log of dilution, where \% infected is over 50\%}) + (\text{proportionality factor x log of dilution factor}).$$

$$\text{Proportionality factor} = [(\text{\% infected over 50\%}) - 50\%] / [(\text{\% infected over 50\%}) - (\text{\% infected below 50\%})].$$

*CPE Inhibition Test. Determination of 50% Inhibitory Concentration (IC50) and Selective Index (SI)*

The antiviral activity of the test substance was determined by the *Mosmann* MTT cell survival test, modified by Shigeta S. for rapid screening of compounds for anti-influenza activity. Under the influence of the cytopathic action of the virus, the cells die. Determination of cell viability by MTT - a test of virus-infected and substance-treated cells, is indicative of the antiviral action of the substance itself.

Twenty-four hour Vero monolayer cultures seeded in 96-well plates ($5 \times 10^3$ cells/well) were inoculated with ten-fold logarithmic dilutions of different HSV-1/HSV-2 strains, ranging from $10^{-1}$ to $10^{-8}$ inclusive, with 4 wells infected with each dilution. The volume of inoculum virus suspension/well was 0.1 mL at an operating dose of 100 $TCID_{50}$. The virus was adsorbed for 1 h in a thermostat at 37 °C, then to each well was added 0.1 mL of growth-supporting nutrient medium. Dilutions of the test substance were done during viral adsorption. After the viral adsorption time elapsed, the plate was treated as follows:

- Cell control (virus-free and untreated cells) - growth-supporting nutrient medium was added to the wells designated for cell control.
- Viral control (infected with virus and untreated cells) - 0.1 mL of growth-supporting nutrient medium was added to the wells designated for virus control.

- Affected cells - (infected with virus and treated with different dilutions of the test substance cells) - 0.1 mL of the previously prepared dilutions of the test substance were dropped into the wells.

Cell cultures were incubated in a thermostat at 37 $\underline{o}$C and 5% CO2 and were observed daily under a light microscope for the development of CPE. The effects of the IPF-H2A impact was recorded afterinfection (p.i.). The infectious titer was calculated by the method of Reed and Muench. After reading the results under a microscope, the medium was decanted, the plates were washed once with 0.2 mL/well of PBS (pH= 7.2), after which the cell survival was determined by the MTT Assay as described above. IPF-H2A activity is expressed as % protection and determined by the formula:

$$\% \ CPE = \frac{[OD_B - OD_{KB}]}{[OD_{KK\Pi} - OD_{KB}]} \times 100$$

Where:

OD$_B$ - Optical density of infected cells treated with the test substance
OD$_{\kappa B}$ - Optical density of virus-infected cells (viral control)
OD$_{\kappa\kappa\Pi}$ - Optical density of uninfected and untreated cells (cell control)

Calculations were performed using the program *GraphPad Prizm* software version 8.00 for Windows. The 50% inhibitory concentration (IC$_{50}$) is the concentration of the test substance that inhibits the virus-induced cytopathic effect by 50%, as determined directly from the dose-response curve.

Based on the ratio of CD$_{50}$ and IC$_{50}$, the selective index (SI) was calculated.

$$SI = CD_{50} / IC_{50}$$

*Statistical Processing of the Obtained Results*

The data obtained are presented with means $\pm$ standard error of the mean (SEM) of at least two separate experiments, each performed in three to five replicates. Statistically significant differences in survival/proliferation between untreated controls and samples treated with different concentrations were determined by one-way analysis of variance ANOVA and subsequent Dunnett's test. Statistically significant differences between untreated controls and treated samples were determined by t-test, with values at $p < 0.05$ being determined as statistically significant.

*Evaluation of the Antiviral Potential of IPF-H2A in vitro*

*Influence of IPF-H2A complex on the survival and proliferative activity of cells, cultured in laboratory environment*

The MTT analysis of cell survival and proliferation was performed in a wide range of concentrations of the test substance. The limits within which the applied concentrations varied were selected on the basis of preliminary experiments involving larger concentration intervals in order to accurately determine the values of CD$_{50}$ and MNC. The test substance was administered in the concentration range 0.0001- 20 mg/mL and the anti-herpes preparation ACV-0.0005- 0.1 mg/mL for 72 h. Untreated Vero cells, whose survival was assumed to be 100%, were used as a negative control. Dose-dependent curves on the viability of the cell monolayer of the applied IPFH2-A were obtained from the MTT Assay used. The analysis during the observation of the cell morphology was synchronized with the MTT analysis of cell survival.

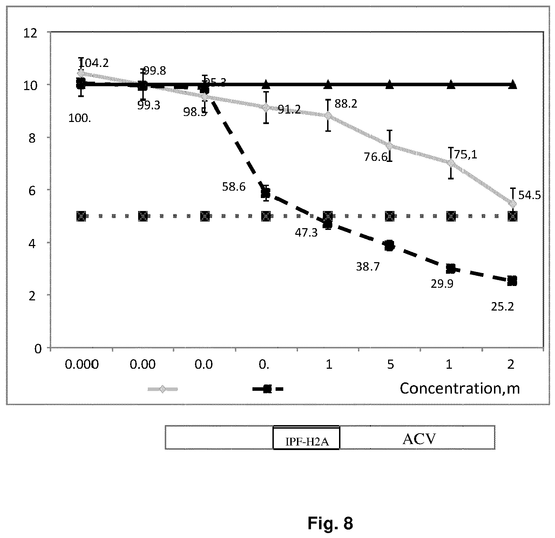

In general, the results show that the survival of the treated Vero monolayer cells did not decrease dramatically under the influence of the inactivated pepsin fragment with Histone Type 2A (IPFH2-A) tested, in contrast to the action of the antiviral drug ACV. As with ACV, the observed effect increases in direct proportion to the increase in its concentration. At the lowest studied incubation concentrations (0.0001- 0.1 mg/mL) relatively weak inhibition of survival was observed (85.34-99.52 %), while at the subsequent increasing concentrations (from 5 mg/mL to 20 mg/mL) the value of cell survival decreases and reaches close to 50%. Survival differences between untreated control and treated sample at doses of 0.0001 mg/mL (p<0.001) and 1 mg/mL (p<0.05) were statistically significant **(Figure** 8).

Based on their cytotoxicity, the test substances are arranged in the following hierarchical order and:

- According to MNC:ACV > IPF-H2A
- According to $CD_{50}$ :ACV> IPF-H2A

When comparing the experimental data on cell survival, it became clear that the widely used anti-herpes preparation ACV is about 30 times more toxic than IPF-H2A.

In conclusion, the tested IPF-H2A does not exhibit toxicity on the cells of the Vero line after 72 h of treatment, as applied in a concentration of 0.0001 mg/mL to 5 mg/mL. The decrease in the number of adherent living cells, as well as the rounding and in the size decrease of the cells follow the trend established during the MTT analysis. Visualization of changes in the morphology of the cell monolayer after treatment is an accessible and reliable approach in the initial study of the cytotoxicity of various substances. In this regard, in parallel with the MTT analysis, changes in the morphology of the cells exposed to the tested IPFH2-Aand the antiviral drug ACV were observed under an inverter light microscope. The non-treated controlcells and the cells treated with IPF-H2A in the concentration range 0.0001-5 mg/mL retain their morphology and structure, while the treated cells with the anti-herpes preparation ACV at the highest concentrations (1-20 mg/mL) acquired a round shape, got reduced in volume and disintegrated from the monolayer.

Effect of IPFH2-A and anti-herpes preparation ACV on survival/proliferative activity ofcells of the Vero line, reported by various methods after 72 h of treatment is summarized on Table 5 below.

Table 5

| IPF-H2A and ACV | Method | MNC (mg/mL) | $CD_{50}$ (mg/mL) |
|---|---|---|---|
| IPF-H2A | Microscopic observation of changes in the morphology of the cell monolayer | $0.1 \pm 0.1$ | $15 \pm 0.05$ |
| | MTT Assay | $0.1 \pm 0.12$ | $15 \pm 0.22$ |
| ACV | Microscopic observation of changes in the morphology of the cell monolayer | $0.05 \pm 0.6$ | $0.5 \pm 0.02$ |
| | MTT Assay | $0.05 \pm 0.07$ | $0.5 \pm 0.1$ |

*Antiviral Activity*

*CPE Inhibition Test*

The IPF-H2A peptide complex was tested for its antiviral effect on HSV-1/HSV-2 in cell cultures.

To test the effects of the IPF-H2A test substance on the HSV-1/HSV-2 replication, concentrations were administered ranging from 0.001 mg/ml to 1 mg/ml (MNC and close to it). The anti-herpes preparation ACV was used for positive control in the antiviral experiments, applied in the concentrationrange of 0.05 - 0.5 mg/mL. Dose-dependent curves were derived to determine the inhibitory concentration leading to 50% inhibition of the viral cytopathic effect ($IC_{50}$).

Initially, the antiviral activity of the IPF test substance was assessed by a CPE inhibition test. After infection of the cells with HSV-1/HSV-2, CPE develops in about 72 hours and resultsin the appearance of round cells, unevenly disseminated in the cell monolayer, with a tendency to form foci and the formation of syncytia. Cell cultures were observed daily under a light microscope as to theCPE development. The effect of the test substances was observed for 72 h (the time for one replicate cycle of coronaviruses) after infection with HSV-1/HSV-2 in Vero cells (p.i.). The results of the experiments were evaluated on the basis of comparison with virus-free and no-added cells (cell control),and with cells infected with HSV-1/HSV-2 without substance addition (viral control). As a positive control, a preparation with proven anti-herpes action ACV was used.

It is clear from the studies that in contrast to uninfected Vero cells, the infected cells with HSV-1/HSV-2 have a characteristic rounded shape, clustered in the form of "islands", isolated from one another and separated from the substrate on which are cultivated.

From the microscopic monitoring of the morphology of the monolayers it can be concluded that the substances tested at the MNC showed varying degrees of inhibition of CPE in HSV-1/ HSV- 2 infected cells after 72 hours of treatment.

The best CPE suppression of HSV-1/HSV-2 was observed after IPF-H2A treatment of infected Vero cells. A less pronounced CPE inhibitory effect but close to that of IPF-H2A was manifested by the antiviral drug ACV. The titer of infectious viral yield decreases significantly when infected Vero cells are treated with IPF-H2A.

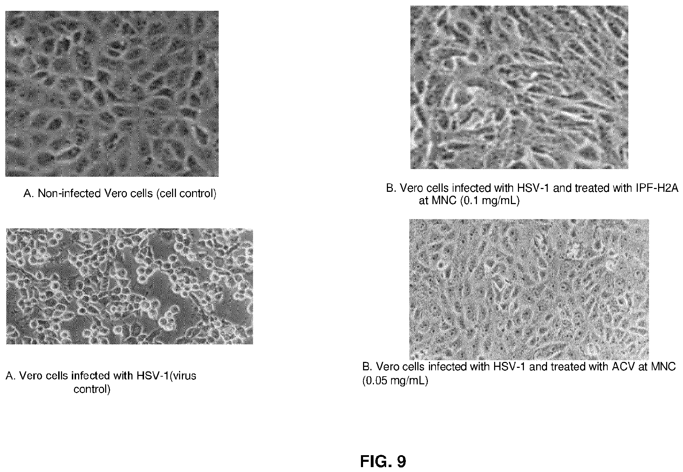

The Inhibitory effects of IPF-H2A and of the anti-herpes preparation ACV, evaluated by CPE inhibition test on HSV-1/HSV-2 infected cells of the Vero line after 72 hours of treatment is shown on **Figure** 9. The test substance and ACV were administered at MNC (mg/mL).

*Determination of the effects of the test substance on infectious viral yield. Determination of 50% inhibitory concentration (IC50) and selective index (SI)*

Concentrations around and close to the minimum effective ones (0.0001-1 mg/mL) were used to determine the anti-HSV-1/HSV-2 activity of the IPF-H2A test substance. Extracellular virus samples were titrated in a 24-hour Vero monolayer in 96-well plates, by taking into account the infectious viral titer in log10, according to the final dilution method of Reed & Muenh. Differences in infectious viral titers between experimental and control samples are expressed as $\Delta$log. Based on the reported results, dose-response graphs were plotted and minimum inhibitory concentrations 50 ($MIC_{50}$) were determined. Values of $\Delta$log10 $\geq$ 1 are considered to be reliable results for the performed tests. Data related to the individual effect of IPF-H2A on the reporting of the infectious titer are summarized in Table 6 below.

Table 6

| Viral strain | Concentration (mg/mL) | Viral yield ($log_{10}$ $TCID_{50}$) | $\Delta$log1 0 |
|---|---|---|---|
| HSV-1 (Victoria strain) | 0 | 5.833 $\pm$ 0.000 | 0 |
| | 0.0 001 | 5.567 $\pm$ 0.288 | 0.8 |
| | 0.0 01 | 4.520 $\pm$ 0.166 | 0.8 |
| | 0.0 1 | 4.500 $\pm$ 0.002 | 1.0 |
| | 0.1 | 4.353 $\pm$ 0.068 | 1.0 |
| | 1 | 4.000 $\pm$ 0.442 | 1.3 |
| HSV-2 (Bja strain) | 0 | 6.000 $\pm$ 0,166 | 0 |
| | 0.0 001 | 5.800 $\pm$ 0.022 | 0 |
| | 0.0 01 | 5.567 $\pm$ 0.288 | 0 |
| | 0.0 1 | 4.500 $\pm$ 0.015 | 0.8 |
| | 0.1 | 4.600 $\pm$ 0.288 | 0.8 |
| | 1 | 4.542 $\pm$ 0.500 | 1.0 |

Based on the data obtained for "dose- anti-HSV-1/HSV-2" it has been established that when IPF-H2A is administered separately, it inhibits the replication of HSV-1/HSV-2 in a dose-dependent manner.

The IPF-H2A complex effectively inhibits the yield of infectious viral progeny in the concentration range of 0.0001- 1 mg/mL. When applied at MNC (0.1 mg/mL) it reduces the viral yield by $\Delta$log10 1.3 and its MIC50 is 0.0001 mg/ml. However, the anti-influenza drug ACV effectively inhibited the replication of HSV-1/HSV-2 in a larger concentration range of 0.00001- 1 mg/mL. The individual effect of the anti-herpes preparation ACV on the reporting of the infectious titer (log10 $TCID_{50}$/ 0.2 ml) is presented in Table 7 below.

The 50% inhibitory concentration ($IC_{50}$) is the concentration of the test substance that inhibits the virus-induced cytopathic effect by 50%, as derived directly from the dose-response curve. Based on the experimental results, the main chemotherapeutic parameter $IC_{50}$ of the tested substances has been reported. The $IC_{50}$ values are an indicator of the antiviral efficacy of a substance, and S! provides information on its specificity and selectivity.

Table 7

| Viral strain | Concentration (mg/mL) | Viral yield (log$_{10}$ TCID$_{50}$) | $\triangle$log$_{10}$ |
|---|---|---|---|
| HSV-1 (Victoria strain) | 0 | 5.300 $\pm$ 0.242 | - |
| | 0.0005 | 4.500 $\pm$ 0.763 | 0.8 |
| | 0.005 | 4.530 $\pm$ 0.166 | 0.8 |
| | 0.05 | 4.300 $\pm$ 0.763 | 1.0 |
| | 0.1 | 4.000 $\pm$ 0.288 | 1.6 |
| | 1 | 2.7 $\pm$ 0.500 | 2.6 |
| HSV-2 (Bja strain) | 0 | 0 | 0 |
| | 0.0005 | 5.567 $\pm$ 0.000 | - |
| | 0.005 | 5.300 $\pm$ 0.288 | 0.8 |
| | 0.05 | 4.500 $\pm$ 0.125 | 1.0 |
| | 0.1 | 4.300 $\pm$ 0.237 | 1.0 |
| | 1 | 4.005 $\pm$ 0.166 | 1.3 |

The total IC$_{50}$ and SI of the tested GP extract and active fraction C according to the MTT Assay are presented in Table 8 below.

Table 8
Antiviral Activity

| | HSV-1 (Victoria strain) | | | HSV-2 (Bja strain) | | |
|---|---|---|---|---|---|---|
| | % cellular protection at MNC | IC$_{50}$ (mg/mL) $\pm$ SEM | SI | % cellular protection at MNC | IC$_{50}$ (mg/mL) $\pm$ SEM | SI |
| IPFH2-A | 90.5 | 0.0001 $\pm$ 0.08 | 1000 | 30.5 | 0.001 $\pm$ 0.03 | 100 |
| ACV | 100 | 0.0005 $\pm$ 0.02 | 800 | 90.7 | 0.005 $\pm$ 0.12 | 80 |

The analysis of the antiviral results has shown that the inhibitory effect of IPFH2-A is selective, strain-specific and dose-dependent. IPFH2-A effectively inhibits the replication of the Victoria strain (HSV-1) at a concentration of 0.0001 mg/mL (with 90.5% cellular protection). In this study the ACV, which was used as a positive control in the concentration range of 0.0005- 0.01 mg/mL has been found to inhibit the replication of the HSV-1 strain Victoria (with 100% complete protection), while the HSV-2 strain Bja has proven to be 10 times less sensitive.

**Example 7:** Antigen- specific therapeutic vaccine based on the IPF-H2A peptide complex

Even as we test these early vaccine approaches in patients, new and more potent vaccine approaches are being developed in preclinical models. These approaches employ tumor-associated antigens either in the form of proteins or peptides, mixed with defined adjuvants administered intradermally or subcutaneously or as genes expressed in viral vectors administered systemically. Antigen-based therapeutic vaccines get rid of the need for the genetic manipulation of tumor cells. This simplifies the vaccine production process and should result in more generalizable vaccines allow for greater control over the amount of antigen formulated in the vaccine, which should translate into improved efficacy. In fact, some of these strategies have been demonstrated in preclinical models to be magnitudes more potent than a whole cell of vaccine approach. Recent advances in molecular biology have allowed the direct isolation of tumor-associated antigens that are the targets of cytotoxic T cells. Tumor associated antigens have now been identified for several cancers including adenocarcinoma of the breast, colon, and pancreas, as well as for malignant melanoma. These antigens can be classified by tissue distribution and function, shown on Table 9. Many of these antigens have been found to be shared among other patients' tumors of the same histologic site. These shared antigens are potential candidates for vaccine development. Several of these antigens are binding *in vitro* or mixed with defined IPF-H2A or pulsed directly onto autologousdendritic cells (Professional APCs) that are activated *in vivo* in each patient. Future immunotherapy of cancer approaches will employ newly identified tumor-associated antigens bind with IPF-H2A that are delivered in patients with different type of cancer. The conduction of well controlled clinical trials will be necessary to identify the most effective approaches for the treatment of advanced cancer. More than likely, it will be necessary to combine vaccine approaches with other cancer treatment as IL-10 cytokines, Th1 MHC class II antigens, and co-stimulatory molecules macrophages modalities to effectively treat larger metastases.

Table 9

| Antigen Category | Human Tumor Antigens |
|---|---|
| Mutated gene products for melanoma | Cyclin-dependent kinase |
| Cell carcinoma of head and neck | Mutated intron B33-B |
| Reactivated silent gene | B-catenin |
| Melanoma | MAGE gene family |
| Melanoma | BAGE gene family |
| Tissue-specific antigens (different antigens) | Tyrosinase (melanoma) Melan-A/MART-1 |
| Melanoma | Gp100 (melanoma) |
| Idio-typic epitopes | Gp75 (melanoma) |
| Viral gene products | Ig (B cell lymphoma) |
| Oncogene products (CML) | TCR (T lymphoma) |
| Products (pancreas and colorectal cancer | EBV (Burkitt's Lymphoma) |
| Overexpression (breast, lung, ovarian cancers) | HPV (cervical cancer) |
| Mutated tumor suppressor | HBV (hepatoma) |
| Products (most cancer) | HER-2/neu |
| Gene products | Mutated p53 gene |

Sequence Listing Information:
        DTD Version: V1_3
        File Name: IPF-H2A complex for modulation immune function.xml
        Software Name: WIPO Sequence
        Software Version: 2.2.0
        Production Date: 2022-12-01
General Information:
        Current application / Applicant file reference: LH_22
        Applicant name: Zhabilov, Harry H.
        Applicant name / Language: en
        Applicant name / Name Latin: Zhabilov, Harry H.
        Inventor name: Zhabilov, Harry H.
        Inventor name / Language: en
        Inventor name / Name Latin: Zhabilov, Harry H.
        Invention title: Peptide complex IPF-H2A for modulation immune function
of the human body ( en )
        Sequence Total Quantity: 3
Sequences:
        Sequence Number (ID): 1
        Length: 31
        Molecule Type: AA
        Features Location/Qualifiers:
                - source, 1..31
                        > mol_type, protein
                        > organism, synthetic construct
        Residues:
        GDEPLENYLD  TEYFNVPVSV  EGYQITLDSI  T
31

        Sequence Number (ID): 2
        Length: 14
        Molecule Type: AA
        Features Location/Qualifiers:
                - source, 1..14
                        > mol_type, protein
                        > organism, Sus scrofa
        Residues:
        GDEPLENYLD  TEYF
14

        Sequence Number (ID): 3
        Length: 17
        Molecule Type: AA
        Features Location/Qualifiers:
                - source, 1..17
                        > mol_type, protein
                        > organism, unidentified
        Residues:
        NVPVSVEGYQ  ITLDSIT
17
END

**Claims**

1. Peptide complex IPF-H2A for modulation immune function of the human body with amino acid sequence SEQ ID: NO. 1, consisting of irreversible pepsin fraction (IPF) and histone type 2A (H2A).

2. Peptide complex IPF-H2A according to claim 1, wherein the irreversible pepsin fraction has amino acid sequence SEQ ID: NO. 2 and the histone type 2A has amino acid sequence SEQ ID NO. 3.

3. Peptide complex IPF-H2A according to claims 1 and 2 for use as an antigen- specific vaccine for immunotherapy.

**FIG. 1**

**Fig. 2**

**Fig. 3**

**Fig. 4**

**Fig. 5**

**Fig. 6**

**Fig. 7**

Fig. 8

A. Non-infected Vero cells (cell control)

B. Vero cells infected with HSV-1 and treated with IPF-H2A at MNC (0.1 mg/mL)

A. Vero cells infected with HSV-1(virus control)

B. Vero cells infected with HSV-1 and treated with ACV at MNC (0.05 mg/mL)

**FIG. 9**

Europäisches
Patentamt
European
Patent Office
Office européen
des brevets

# EUROPEAN SEARCH REPORT

Application Number

EP 22 02 0605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2009/285776 A1 (ZHABILOV HARRY H [US]) 19 November 2009 (2009-11-19) * paragraph [0081] * | 1-3 | INV. A61K39/39 C07K14/47 A61P31/12 A61K38/10 C07K7/06 |
| Y | US 2006/104992 A1 (ZHABILOV HARRY H [US]) 18 May 2006 (2006-05-18) * paragraph [0010] * | 1-3 | |
| Y | AGAZIO AMANDA ET AL: "Histone H2A-Reactive B Cells Are Functionally Anergic in Healthy Mice With Potential to Provide Humoral Protection Against HIV-1", FRONTIERS IN IMMUNOLOGY, vol. 11, 1 January 2020 (2020-01-01), page 1565, XP093044947, DOI: 10.3389/fimmu.2020.01565 * abstract * | 1-3 | |
| Y | GUSTAVO DOMÍNGUEZ-BERNAL ET AL: "Mitigating an undesirable immune response of inherent susceptibility to cutaneous leishmaniosis in a mouse model: the role of the pathoantigenic HISA70 DNA vaccine", VETERINARY RESEARCH, BIOMED CENTRAL LTD, LONDON, UK, vol. 43, no. 1, 9 August 2012 (2012-08-09), page 59, XP021119817, ISSN: 1297-9716, DOI: 10.1186/1297-9716-43-59 * abstract * | 1-3 | TECHNICAL FIELDS SEARCHED (IPC) A61K C07K A61P C12N |
| A | CHEW GUO-LIANG ET AL: "Short H2A histone variants are expressed in cancer", NATURE COMMUNICATIONS, vol. 12, no. 1, 20 January 2021 (2021-01-20), XP093044973, DOI: 10.1038/s41467-020-20707-x p 7, col 2, first lines; * abstract * | 1-3 | |

The present search report has been drawn up for all claims

| Place of search | Date of completion of the search | Examiner |
|---|---|---|
| Munich | 10 May 2023 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 02 0605

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2023

| Patent document cited in search report | | Publication date | Patent family member(s) | | Publication date |
|---|---|---|---|---|---|
| US 2009285776 | A1 | 19-11-2009 | NONE | | |
| US 2006104992 | A1 | 18-05-2006 | CA | 2634589 A1 | 22-06-2006 |
| | | | EP | 1830867 A2 | 12-09-2007 |
| | | | US | 2006104992 A1 | 18-05-2006 |
| | | | US | 2010204133 A1 | 12-08-2010 |
| | | | WO | 2006066065 A2 | 22-06-2006 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040018639 A
- EP 1830867 A2
- US 7479538 B2, Zhabilov, H.

### Non-patent literature cited in the description

- *Nature,* 1997, vol. 387, 426-30
- *Nature,* 1998, vol. 393 (6686), 632-1
- *Molecular Membrane Biology,* 1999, vol. 16, 3-9
- **WILLIAMS, W. M.** *Clin Exp Immunol,* 1996, vol. 104, 18-24
- Thymus Dependency. **BACH, J.F.** Contemporary Topics in Immuno Biology. Plenum Press, 1973, vol. 2, 189
- **HOFFMAN, T. ; KUNKEL, H. G. ; KAPLAN, M. E. et al.** In Vitro Metlzo & in Cell Mediated and Tumor Immunity. Academic Press, 1976